Europäisches Patentamt

European Patent Office

Office européen des brevets

(11) Publication number: **0 331 356**
**A2**

(12) **EUROPEAN PATENT APPLICATION**

(21) Application number: **89301803.6**

(22) Date of filing: **23.02.89**

(51) Int. Cl.4: **C12N 15/00 , C12N 1/20 , C12N 1/18 , C12P 21/02 , A61K 37/02 , //(C12N1/20, C12R1:19,1:465)**

Claims for the following Contracting State: ES.

(30) Priority: **24.02.88 US 160463**

(43) Date of publication of application:
**06.09.89 Bulletin 89/36**

(84) Designated Contracting States:
**AT BE CH DE ES FR GB GR IT LI LU NL SE**

(71) Applicant: **SMITHKLINE BECKMAN CORPORATION**
**P.O. Box 7929 1 Franklin Plaza**
**Philadelphia Pennsylvania 19101(US)**

(72) Inventor: **Arthos, James**
**2026 Hill Street**
**Ann Arbor Michigan 48104(US)**
Inventor: **Clark, Philip Ernest**
**12 Donna Lane**
**Wayne Pennsylvania 19087(US)**
Inventor: **Fornwald, James Allan**
**104 Burnside Avenue**
**Norristown Pennsylvania 19403(US)**

Inventor: **Brawner, Mary Ellen**
**126 Valley Stream Circle**
**Wayne Pennsylvania 19087(US)**
Inventor: **Deen, Keith Charles**
**21 Maude Circle**
**Paoli Pennsylvania 19301(US)**
Inventor: **Gorman, Jessica Angell**
**152 Ridgefield Road**
**Newtown Square Pennsylvania 19073(US)**
Inventor: **Sathe, Ganesh Madhusudan**
**412 Hillside Road**
**King of Prussia Pennsylvania 19406(US)**
Inventor: **Sweet, Raymond Whitney**
**108 Edgehill Road**
**Bala Cynwyd Pennsylvania 19004(US)**
Inventor: **Taylor, Dean Perron**
**400 Hillside Road**
**King of Prussia Pennsylvania 19406(US)**

(74) Representative: **Giddings, Peter John, Dr. et al**
**Smith Kline & French Laboratories Ltd.**
**Corporate Patents Mundells**
**Welwyn Garden City Hertfordshire AL7 1EY(GB)**

(54) **Expression of HIV binding proteins.**

(57) The expression of HIV binding proteins derived from the CD-4 receptor protein in recombinant host cells such as E.coli, Streptomyces and yeast, and novel vectors used in the expression of said proteins.

EP 0 331 356 A2

## Expression of HIV Binding Proteins

### Field of the Invention

This invention relates to production of HIV binding proteins derived from soluble CD-4 (sCD-4) proteins. More particularly it relates to the construction of vectors for the expression of HIV binding proteins in E. coli, Streptomyces and yeast.

### Background of the Invention

The human T-cell glycoprotein, CD-4 (sometimes previously referred to as "T4"), is one of several non-polymorphic T lymphocyte surface receptor proteins that have been implicated in the mediation of efficient T cell-target cell interactions.

The cDNA sequence of the human CD-4 (T-4) receptor is known (Maddon,et al., Cell 42:93(1985)). The complete CD-4 pre-protein sequence is 458 amino acids in length comprising the putative 23 amino acid secretory leader, 372 amino acid surface ($V_1$-$V_4$), 23 amino acid transmembrane and 40 amino acid cytoplasmic domains. The surface domain shows four regions of limited homology, 20-30%, to immunoglobulin variable (V) and joining (J) regions. Four of the five intron-exon boundaries in the surface domain occur near the junctions of these V-J regions.

The human immunodeficiency virus (HIV), the etiological agent of acquired immune deficiency syndrome (AIDS), shows a marked affinity for CD-4 lymphocytes. Virus binding to CD-4 is mediated by the gp120 glycoprotein of the viral envelope. The CD-4 protein can be co-precipitated from lysed HIV infected cells with anti-env antibody and, conversely, gp120 can be co-precipitated with anti-CD-4 monoclonal antibody, thus demonstrating that virus binding to CD-4 is mediated by the gp120 glycoprotein of the viral envelope.

Recently, a number of scientific investigators have reported their findings related to the interaction between CD-4 and the AIDS infection using a CD4 derivative expressed in mammalian cell systems.

Deen, et al., Nature 331:82(1988) report the isolation and expression of sCD-4 in several cellular environments. sCD-4 is a soluble derivative of CD-4 which lacks the transmembrane and cytoplasmic domains of CD-4. Using a dfhr deficient Chinese Hamster Ovary (CHO) cell line they have shown the expressed sCD-4 retains the structural and biological properties of CD-4 on the cell surface, binds to the HIV gp 110 envelope protein and can inhibit the binding of CD-4$^+$ lymphocytes.

Traunecker, et al., Nature 331:84(1988) report use of sCD-4 to inhibit the attachment of virus to the target cell. The data presented shows the inhibition of HIV infection in vitro using a recombinant form of sCD-4.

Fisher, et al., Nature 331:76(1988) report the purification of sCD-4 from (CHO) and its use as a potent inhibitor of both virus replication and virus induced cell fusion.

Hussey, et al., Nature 331:78(1988) report the use of the baculovirus expression system to produce sCD-4 which was found to inhibit virus induced cell fusion (syncytium formation) and HIV infection by binding to gp120 expressing cells. It was also shown that the sCD-4 protein does not appear to inhibit class II specific T-cell interactions.

Smith, et al., Science 238:1704(1987) also report use of the CHO mammalian cell system to produce sCD-4. They report the production of a sCD-4 protein in CHO cells which can be shown to bind to HIV gp 120 envelope protein and neutralize the infectivity of HIV-1 in vitro. The data indicate that sCD-4 can be used as a basis for therapeutic treatment of AIDS infection.

There is still a need, however, for bacterial and lower eukaryotic systems for expression of s-CD4 proteins.

### Summary of the Invention

This invention lies in the discovery that the HIV binding function of the CD-4 protein can be expressed in E. coli and in the discovery that such HIV binding protein can be secreted into the fermentation medium.

In particular, therefore, this invention is an E. coli expression vector comprising an HIV binding protein operatively linked to a regulatory element.

This invention also lies in the discovery that the HIV binding function of the CD-4 protein can be expressed in Streptomyces and in the discovery that such HIV binding protein can be secreted into the fermentation medium. In particular, therefore, this invention is a Streptomyces expression vector comprising an HIV binding protein operatively linked to a regulatory element.

This invention also lies in the discovery that the HIV binding function of the CD-4 protein can be expressed in yeast. In particular, therefore, this invention is a yeast expression vector comprising an HIV binding protein operatively linked to a regulatory element.

This invention also lies in the discovery that the OMPA leader sequence can be used to export heterologous proteins from E. coli. In particular, therefore, this invention is an E. coli expression vector for expressing exported proteins in E. coli comprising a DNA coding sequence operatively linked to a regulatory element wherein the DNA coding sequence codes for the protein X-Y wherein X is an OMPA leader sequence and Y is a heterologous protein.

## Detailed Description of the Invention

In accordance with the present invention, HIV binding proteins derived from the CD-4 receptor are expressed in useful quantities for use as therapeutic and/or prophylactic agents to inhibit initial HIV infection and to inhibit cell-to-cell transmission of HIV following host infection; for use in screens for inhibitors of HIV-lymphocyte binding; for use as inhibitors of CD-4$^+$ lymphocyte functions; or for other uses such as described below. The proteins all have the HIV binding function from CD-4. Preferred examples of such proteins are sCD-4 proteins and derivatives thereof. sCD-4 proteins are derivatives of CD-4 which lack the transmembrane and cytoplasmic domains of CD-4. Examples of sCD-4 proteins are described in separate papers by Deen et al., Traunecker et al., Fisher et al., and Hussey et al., in Nature 331:76-88 (1988) and by Smith et al., Science 238:1704(1987).

The sCD-4 protein exemplified herein, referred to as SKBsCD-4, is reported by Deen et al., cited above, at 83. It is shown in the following amino acid and nucleotide sequence. The sequence shows a 5' untranslated region and a leader sequence derived from the CD-4 cDNA reported by Maddon, et al., Cell 42:93(1985). The first amino acid of mature SKBsCD-4 as expressed and secreted by CHO cells is the lysine encoded by base pairs 151-153. The transmembrane domain was deleted by restricting CD-4 cDNA with HpaII at base pair 1252 and ligating thereto a linker which restored base pairs 1253-1257 and which placed a translation stop codon, TAA, after the C-terminal valine. As reported by Maddon, et al., the first three amino acids of mature CD-4 are glutamine (shown herein as amino acid (-)2) - glycine (shown here as amino acid (-)1) -asparagine (shown here as lysine, amino acid (+)1). Otherwise, the sequence reported below is the same as that reported by Maddon et al.

```
                10                  30                  50
CAAGCCCAGAGCCCTGCCATTTCTGTGGGCTCAGGTCCCTACTGCTCAGCCCCTTCCTCC


                70                  90                 110
CTCGGCAAGGCCACAATGAACCGGGGAGTCCCTTTTAGGCACTTGCTTCTGGTGCTGCAA
                  MetAsnArgGlyValProPheArgHisLeuLeuLeuValLeuGln


               130                 150                 170
CTGGCGCTCCTCCCAGCAGCCACTCAGGGAAAGAAAGTGGTGCtGGGCAAAAAAGGGGAT
LeuAlaLeuLeuProAlaAlaThrGlnGlyLysLysValValLeuGlyLysLysGlyAsp
    -9                          -1  +1


               190                 210                 230
ACAGTGGAACTGACCTGTACAGCTTCCCAGAAGAAGAGCATACAATTCCACTGGAAAAAC
ThrValGluLeuThrCysThrAlaSerGlnLysLysSerIleGlnPheHisTrpLysAsn


               250                 270                 290
TCCAACCAGATAAAGATTCTGGGAAATCAGGGCTCCTTCTTAACTAAAGGTCCATCCAAG
SerAsnGlnIleLysIleLeuGlyAsnGlnGlySerPheLeuThrLysGlyProSerLys


               310                 330                 350
CTGAATGATCGCGCTGACTCAAGAAGAAGCCTTTGGGACCAAGGAAACTTCCCCCTGATC
LeuAsnAspArgAlaAspSerArgArgSerLeuTrpAspGlnGlyAsnPheProLeuIle


               370                 390,                410
ATCAAGAATCTTAAGATAGAAGACTCAGATACTTACATCTGTGAAGTGGAGGACCAGAAG
IleLysAsnLeuLysIleGluAspSerAspThrTyrIleCysGluValGluAspGlnLys


               430                 450                 470
GAGGAGGTGCAATTGCTAGTGTTCGGATTGACTGCCAACTCTGACACCCACCTGCTTCAG
GluGluValGlnLeuLeuValPheGlyLeuThrAlaAsnSerAspThrHisLeuLeuGln
                                                   104


               490                 510                 530
GGGCAGAGCCTGACCCTGACCTTGGAGAGCCCCCCTGGTAGTAGCCCCTCAGTGCAATGT
GlyGlnSerLeuThrLeuThrLeuGluSerProProGlySerSerProSerValGlnCys


               550                 570                 590
AGGAGTCCAAGGGGTAAAAACATACAGGGGGGGAAGACCCTCTCCGTGTCTCAGCTGGAG
ArgSerProArgGlyLysAsnIleGlnGlyGlyLysThrLeuSerValSerGlnLeuGlu


               610                 630                 650
CTCCAGGATAGTGGCACCTGGACATGCACTGTCTTGCAGAACCAGAAGAAGGTGGAGTTC
LeuGlnAspSerGlyThrTrpThrCysThrValLeuGlnAsnGlnLysLysValGluPhe
151


               670                 690                 710
AAAaTAGACATCGTGGTGCTAGCTTTCCAGAAGGCCTCCAGCATAGTCTATAAGAAAGAG
LysIleAspIleValValLeuAlaPheGlnLysAlaSerSerIleValTyrLysLysGlu
                                                   183
```

4

```
                    730                       750                       770
GCGGAACAGGTGGAGTTCTCCTTCCCACTCGCCTTTACAGTTGAAAAGCTGACGGGCAGT
GlyGluGlnValGluPheSerPheProLeuAlaPheThrValGluLysLeuThrGlySer


                    790                       810                       830
GGCGAGCTGTGGTGGCAGGCGGAGAGGGCTTCCTCCTCCAAGTCTTGGATCACCTTTGAC
GlyGluLeuTrpTrpGlnAlaGluArgAlaSerSerSerLysSerTrpIleThrPheAsp


                    850                       870                       890
CTGAAGAACAAGGAAGTGTCTGTAAAACGGGTTACCCAGGACCCTAAGCTCCAGATGGGC
LeuLysAsnLysGluValSerValLysArgValThrGlnAspProLysLeuGlnMetGly


                    910                       930                       950
AAGAAGCTCCCGCTCCACCTCACCCTGCCCCAGGCCTTGCCTCAGTATGCTGGCTCTGGA
LysLysLeuProLeuHisLeuThrLeuProGlnAlaLeuProGlnTyrAlaGlySerGly


                    970                       990                      1010
AACCTCACCCTGGCCCTTGAAGCGAAAACAGGAAAGTTGCATCAGGAAGTGAaCCTGGTG
AsnLeuThrLeuAlaLeuGluAlaLysThrGlyLysLeuHisGlnGluValAsnLeuVal


                   1030                      1050                      1070
GTGATGAGAGCCACTCAGCTCCAGAAAAATTTGACCTGTGAGGTGTGGGGACCCACCTCC
ValMetArgAlaThrGlnLeuGlnLysAsnLeuThrCysGluValTrpGlyProThrSer


                   1090                      1110                      1130
CCTAAGCTGATGCTGAGCTTGAAACTGGAGAACAAGGAGGCAAAGGTCTCGAAGCGGGAG
ProLysLeuMetLeuSerLeuLysLeuGluAsnLysGluAlaLysValSerLysArgGlu


                   1150                      1170                      1190
AAGGCGGTGTGGGTGCTGAACCCTGAGGCGGGGATGTGGCAGTGTCTGCTGAgTGACTCG
LysAlaValTrpValLeuAsnProGluAlaGlyMetTrpGlnCysLeuLeuSerAspSer


                   1210                      1230                      1250
GGACAGGTCCTGCTGGAATCCAACATCAAGGTTCTGCCCACATGGTCCACCCCCGtgtaa
GlyGlnValLeuLeuGluSerAsnIleLysValLeuProThrTrpSerThrProVal End
351                                                              369


                   1270
tggcggcctctaga
```

sCD-4 truncates, and derivatives thereof, which substantially retain the HIV binding affinity of sCD-4 can also be expressed in accordance with this invention. Preferred among such truncates are proteins or polypeptides comprising the HIV binding region of the V1 and V2 regions which is comprised approximately within amino acids 1-182. With reference to the sequence shown above, one particular example is the protein herein referred to as V1J4 which has amino acids (-)2-104 (N-glutamine-glycine-lysine- through -alanine-asparagine-serine-C) fused to amino acids 351-369 (-glycine-glutamine -valine through -threonine-proline-valine-C). A second example is the protein herein referred to as V1V2J4 which has amino acids (-)2-183 (N-glutamine-glycine-lysine-through -lysine-alanine-serine-C) fused to amino acids 351-369. A third example is the protein herein referred to as YV1, which comprises amino acids (-)9-151 (N-alanine-leucine-leucine- through -leucine-glutamic acid-leucine-C). A fourth example is the protein herein referred to as SKBYsCD-4 which has amino acids (-)9-369. Each of these HIV binding proteins, based on, e.g., OKT4A binding, has the HIV binding property of sCD-4 proteins, specifically of SKBsCD-4, and, more particularly, has the HIV binding domain of the V1 and V2 regions of sCD-4 proteins, specifically of SKBsCD-4.

In addition to those proteins specifically exemplified herein, one of ordinary skill in the art can readily construct DNA coding sequences for further sCD-4 derivatives. Such derivatives substantially retain the HIV binding function of sCD-4 proteins, i.e., the binding of the protein to the HIV env protein, gp120, is not significantly adversely affected. For example, one can construct a derivative having only the V1 region, e.g., approximately amino acids 1-94, or a derivative having only a portion of the V1 region of CD-4. One can also construct derivatives of the proteins specifically exemplified herein, such as derivatives differing by one or a few amino acid deletions, additions or substitutions.

Alternatively, a hybrid protein, i.e., a translational fusion, can be constructed between a sCD-4 protein and a protein carrier, another antigen or other sCD-4 molecules to prepare a poly-sCD-4 molecule. In yet another alternative, the sCD-4 deletion mutant can be synthetically conjugated to a carrier molecule.

Affinity of the sCD-4 molecules for HIV can be demonstrated by a competitive binding assay using a sCD-4 molecule having a known affinity or using antibodies which recognize the CD-4 receptor, such as OKT-4 and OKT4A. Useful sCD-4 proteins of the invention are selectively precipitated from conditioned medium by OKT4A as shown in the Examples, below. CD-4, fragments and derivatives can be prepared chemically, after expression, or genetically, prior to expression, by manipulation of the coding sequence for the leader and/or extracellular domain.

A DNA coding sequence for sCD-4 or HIV binding protein derived therefrom can be obtained, for example, by synthesizing the gene using the known DNA sequence, by standard cloning techniques based on the sequence and by reisolation by detection of protein, i.e., transfection of cDNA clones from CD-4 expressing cell lines and identification by antibodies directed against the protein (See Maddon, et al., supra).

cDNA clones carrying the coding sequence of the particular sCD-4 protein can be identified by use of oligonucleotide hybridization probes. The probes can be designed based on the particular sequence of the protein. Having identified a clone carrying the coding sequence of interest, the coding sequence of the protein can be excised by the use of restriction endonucleases and inserted into cloning and/or expression vectors. In an expression vector, the coding sequence of the sCD-4 protein is operatively linked to regulatory functions required or desirable for transcription, translation and processing of the coding sequence.

In carrying out the present invention in E. coli, a DNA coding sequence which encodes the HIV binding protein, e.g., sCD-4 or derivatives thereof, is operatively linked to a regulatory element within a DNA vector for transformation of E. coli. Numerous gram negative bacterial expression vectors comprising such regulatory elements are available. The regulatory element comprises a promoter which effects RNA polymerase binding and transcription. Regulatable, e.g., inducible or derepressible, promoters are preferred. A variety of useful promoters are available for expression of heterologous polypeptides in E. coli. These include the trp promoter and the lambda PL promoter. See, e.g., U.S. Patent 4,578,355; Courtney et al., Nature 313:149 (1985). Most preferably, the regulatory element comprises a leader sequence for transport of the HIV binding protein to or through the cell membrane as it has been discovered in accordance with this invention that the HIV binding protein can be secreted. A leader which has been shown to transport a protein to the E. coli outer membrane is that of the Bacillus licheniformis penicillinase gene. See, Sarvas, et al., J. Bacteriol. 155:657(1983).

Particularly exemplified herein is the leader for the outer membrane polysaccharide A, herein referred to as the OMPA leader or signal. See, Movva, et al., J. Molec. Biol. 143:317(1980). The OMPA coding sequence comprises a leader sequence which is useful for secreting the HIV binding protein into the fermentation medium. It has now been found that the OMPA leader can be used to fully secrete a variety of low molecular weight proteins or polypeptides into the culture media. For example, the OMPA signal has been used to obtain export of large amounts (e.g., more than 1 to about 10 mg/L) of active human transforming growth factor-alpha (TGF-alpha), tumor necrosis factor (TNF) and interleukin-1-beta (IL-1-beta), in addition to SKBsCD-4, V1J4 and V1V2J4.

The OMPA signal comprises substantially the following 21 amino acids: N-met-lys-lys-thr-ala-ile-ala-ile-ala-val-ala-leu-ala-gly-phe-ala-thr-val-ala-gln-ala-C. A DNA coding sequence for such leader can be synthesized, preferably such that one or more restriction sites are placed adjacent the C-terminal amino acid for insertion of a desired DNA coding sequence and such that a terminator is placed downstream of the desired coding sequence. An illustrative synthetic coding sequence is shown in the Examples, below.

6

In addition to the HIV binding protein minigene, i.e., coding sequence operatively linked to a regulatory element, the vector used for transformation preferably contains at least one phenotypic transformation marker, i.e., genetic selection marker, such as an antibiotic resistance gene, a chromogenic agent or a gene which complements an auxotrophic mutation. The vector also comprises regions required for autonomous replication.

Illustrative constructions of useful expression vectors for expression of sCD-4 proteins in E. coli are described in detail in the Examples. The proteins were expressed into the medium and were apparently folded properly.

In carrying out the present invention in Streptomyces, a DNA sequence which encodes the HIV binding protein, e.g., sCD-4 or derivatives thereof, is operatively linked to a regulatory element within a DNA vector for transformation of Streptomyces. The regulatory element comprises a promoter which effects RNA polymerase binding and transcription. Regulatable, i.e., inducible or derepressible, promoters are preferred. A variety of useful promoters are available for expression of heterologous polypeptides in Streptomyces. Examples include the galactose-inducible promoter of the Streptomyces galactose operon (Fornwald, et al., Proc. Natl. Acad. Sci. USA 84:2130 (1987)), the constitutive promoter of the S. lividans β-galactosidase gene (Eckhardt, et al. J. Bacteriol. 169:4249 (1987); Brawner, et al., U.S. Patent 4,717,666) and the S. longisporus trypsin inhibitor gene (European Patent Application No. 87 307 260.7), or a temporally regulated promoter such as that reported in M. echinosporsa (Baum, et al., J. Bacteriol. 170:71 (1988)). Regions for transcription termination in Streptomyces are derived from the 3′ end of several Streptomyces genes, for example the termination signal at the end of the Streptomyces galactose operon or that found at the end of the S. fradiae neomycin phosphotransferase gene (Thompson and Gray, Proc. Natl. Acad. Sci USA 80:5190 (1983)). Sequences for protein export in Streptomyces include those isolated from the S. lividans β-galactosidase gene, the S. lividans LEP-10 gene (European Patent Application No. 87 307 260.7) and the S. longisporus trypsin inhibitor gene.

The sCD-4 gene is incorporated into a larger DNA molecule which comprises a genetic selection marker system. The selection marker system can be any of a number of known marker systems such that the marker gene confers a selectable new phenotype on the transformed cell. Examples include Streptomyces drug resistance genes such as thiostrepton resistance ribosomal methylase (Thompson, et al., Gene 20:51(1982)), neomycin phosphotransferase (Thompson, et al., supra) and erthromycin resistance ribosomal methylase (Thompson, et al., supra). The DNA molecule may also contain a sequence for autonomous replication in Streptomyces, such as the pIJ101 derivatives (Keiser, et al., Mol. Gen. Genet. 185:223 (1982)) or an SLP1 derived vector (Bibb, et al., Mol. Gen. Genet. 184:230 (1981)). The DNA molecule may also contain a marker which permits gene amplification. Such markers which serve to amplify gene copy number in Streptomyces include the gene for spectinomycin resistance (Hornemann, et al. J. Bacteriol. 169:2360 (1987)) and arginine axuotrophy (Altenbuchner, et al., Mol. Gen. Genet. 195:134 (1984)).

For the initial expression studies, the CD-4 coding sequence as more fully described in the Examples, below, was fused to the βgal signal sequence at a position located 8 amino acids downstream of the βgal signal peptide cleavage site. The Streptomyces replication functions for this expression vector was provided by plasmid pIJ702 (Katz, et al., J. Gen. Microbiol. 129:2703(1983)), which is a derivative of pIJ101 (Keiser, et al., Mol. Gen. Genet. 185:223(1982)). The host strain used was wild-type S. lividans 1326 (Bibb, et al., Mol. Gen. Genet. 184:230(1981)). The βgal-sCD-4 fusion protein was detected in both the culture supernatant and intact cells. Another series of experiments showed that the LTI promoter could be used to obtain more efficient expression of the sCD-4 proteins.

The LTI expression/secretion vectors are analogous to the βgal system in that gene expression is direction by the LTI promoter and protein export is facilitated by the LTI signal peptide. Coding sequences for a number of HIV binding proteins have been cloned into the LTI vector including $V_1V_2J_4$ and $V_1J_4$. The coding sequences for all of these proteins are inserted at a position located 8 amino acids downstream of the LTI signal peptide cleavage site. Like the βgal fusions, the sCD-4 proteins can be transported into the culture supernatant via the LTI signal peptide. The Streptomyces replication functions were again provided by pIJ101 derivatives: pIJ702 and pIJ351 (Keiser, et al., supra).

SKBsCD-4, V1J4 and $V_1V_2J_4$ expression from the LTI expression system was initially examined in S. lividans 1326. Both SKBsCD-4 and $V_1V_2J_4$ were equally distributed between the culture supernatant and intact cells. Although these proteins are incompletely exported, the intra- and extracellular forms appear to have the same molecular weight as judged by their mobilities on SDS-polyacrylamide gels. It is unclear if the signal peptide is proteolytically removed from the intracellular form. The maximum levels of SKBsCD-4 were estimated to be 30 mg/l in the culture supernatant and intact cells. The $V_1V_2J_4$ levels were estimated to be at least 30 mg/l (culture supernatant and intact cells).

One problem encountered for sCD-4 expression in S. lividans is its susceptibility to protease activities.

This problem can be eliminated or at least significantly diminished by expressing sCD-4 in other Streptomyces species. The sCD-4 proteins can be expressed, for example, in S. coelicolor, S. longisporus and S. albus. It is expected the stability of the molecule will be enhanced using these alternate hosts. Expression of sT4 proteins in S. longisporus may be advantageous since this host secretes comparatively fewer proteins into the culture supernatant which assists in the purification process.

In carrying out the present invention in yeast, a DNA coding sequence which encodes the HIV binding protein, e.g., sCD-4 or derivatives thereof, is operatively linked to a regulatory element within a DNA vector for transformation of yeast. Any yeast host for which transformation, cloning and expression systems are available can be used. Particular examples include yeasts of the genera Hansenula, Pichia, Kluveromyces, Schizosaccharomyces, Candida and Saccharomyces. The preferred yeast host is Saccharomyces cerevisiae.

The regulatory element comprises a promoter which effects RNA polymerase binding and transcription. Regulatable, i.e., inducible or derepressible, promoters are preferred. A variety of useful promoters are available for expression of heterologous polypeptides in yeast. These include the copper inducible metallothionein gene (CUP1) promoter and the constitutive promoter of the glycolytic genes glyceraldehye-3 phosphate dehydrogenase (TDH3) and alcohol dehydrogenase (ADH). Regions for transcriptional termination in yeast are derived from the 3' end of any of several yeast genes, for example the gene for iso-1-cytochrome C (CYC1).

The sCD-4 gene is incorporated into a larger DNA molecule which comprises a genetic selection marker system. The selection marker system can be any of a number of known marker systems, such that the marker gene confers a selectable new phenotype on the transformed cell. Examples include yeast genes for biosynthetic enzymes such as phospho-ribosyl anthranilate isomerase (TRP1) or orotidine-5' - phosphate decarboxylase (URA3) or heterologous drug resistance genes such as G418 resistance or benomyl resistance (BEN1). The DNA molecule may also contain a sequence for autonomous replication in yeast, such as the yeast 2-micron-circle ori region or a chromosomal autonomous replication region (ARS), such as ARS1, and a yeast centromere (CEN), such as CEN3, to allow for autonomous replication of the plasmid.

In the preferred expression system, the HIV binding protein coding sequence is fused to a coding sequence which stabilizes expression, inasmuch as it has been discovered that SKBsCD-4 alone expresses poorly; such poor expression may be due to inefficient transcription and translation or to rapid protein degradation. In any case, it has been demonstrated that the product of a gene fusion, in which the coding sequence of a ubiquitin is fused 5' to the SKBsCD-4 coding sequence is expressed at relatively high levels and is cleaved to yield ubiquitin and the sCD-4 gene product.

A ubiquitin DNA coding sequence can be isolated from yeast or other eukaryotic cell, including mammalian cells, by standard gene cloning techniques. Alternatively, it can be synthesized by standard known techniques. The nucleotide sequence of the modular ubiquitin gene used to illustrate the instant invention is that described by Ecker et al., J. Biol. Chem. 262:3524 (1987). The sequence is substantially as follows:

AATTCATTATGCAGATCTTCGTCAAGACGTTAACCGGTAAAACCATAACTCTAGA

AGTTGAATCTTCCGATACCATCGACAACGTTAAGTCGAAAATTCAAGACAAGGAA

GGCATTCCACCTGATCAACAAAGATTGATCTTTGCCGGTAAGCAGCTCGAGGACG

GTAGAACGCTGTCTGATTACAACATTCAGAAGGAGTCGACCTTACATCTTGTCTT

AAGACTAAGAGGTGGTTGAGGTAC

By inserting a DNA sequence for a signal peptide at the 5' end of the HIV binding protein sequence, secretion of protein into the culture medium can be achieved. Signal sequences useful for this purpose are, for example, those of the yeast mating factor, alpha-factor (Kurjan and Herskowitz, Cell 30:933(1982). In addition, expression may be obtained by integration of the sCD-4 yeast expression module into the yeast chromosome, followed by amplification, using markers such as the yeast DHFR gene to select for increased copy number.

In general, the sCD-4 proteins of the invention can be purified from spent culture media using various protein purification techniques, for example, affinity chromatography; ion exchange chromatography; size exclusion chromatography; hydrophobic chromatography or reversed phase chromatography.

sCD-4 its fragments and derivatives can be purified by affinity chromatography using general group-specific adsorbents, for example, carbohydrate binding or dye affinity ligands; or using ligands that specifically bind to sCD-4, for example, monoclonal antibody or HIV gp120 protein or portions thereof.

Purification comprises, after growing cells in a selective growth media at about 28 to about 45° C: clarification of the fermentation medium and then separation of the HIV binding protein from other proteins present in the media. In the preferred method, the sCD-4 proteins are purified from culture medium using a series of chromatography steps which are based on the physical properties of the sCD-4 molecule.

An alternative method of purification of sCD-4 proteins involves the use of monoclonal antibodies directed against sCD-4. The protein can be purified in one step by passage of clarified culture media through an affinity gel support to which monoclonal antibody directed against the protein is bound. The protein of interest will bind to the column at the antibody binding site while contaminating proteins wash through the column. The protein is then eluted from the column under conditions that prevent inactivation.

Characterization of the in vitro biological and immunological properties of the sCD-4 proteins indicate the proteins are of value in the prevention and treatment of AIDS. Studies indicate the sCD-4 proteins act as an inhibitor of extracellular and cell to cell spread of the virus. Because sCD-4, fragments and derivatives thereof described in this invention have been shown to block virus binding to CD-4[+] target cells in culture, it is believed administration of these proteins to infected persons would act to inhibit the extracellular spread of the virus. Therefore, sCD-4 proteins are of value both as a prophylactic and therapeutic agent for treatment of AIDS or AIDS-related complex (ARC).

As a prophylactic, sCD-4 proteins are administered to individuals at high-risk for the disease or individuals who show exposure to HIV by the presence of antibodies to virus. Administration of an effective amount of the protein at an early stage of the disease or prior to its onset acts to inhibit infection of CD-4[+] lymphocytes by HIV. As a therapeutic, administration of sCD-4 to persons infected with HIV acts to inhibit extracellular spread of the virus.

Fusion between HIV infected cells and other CD-4[+] lymphocytes also appears to be a route of virus spread. Further, fusion may be responsible, in part, for the impairment of CD-4[+] lymphocyte function and ultimately the depletion of CD-4[+] lymphocytes in infected individuals. Cell fusion is dependent on both viral envelope gene products and the CD-4 receptor and can be inhibited by the OKT-4A, or similar monoclonal antibodies (Mabs) (Sattentau, et al., Science 234:1120(1986)). sCD-4 proteins described herein interfere with cell fusion and therefore diminish the cell to cell spread of virus and the loss of CD-4[+] lymphocyte function.

The CD-4 receptor is monomorphic and thus sCD-4 proteins are believed to be a universal inhibitor of virus which recognize the surface domain of the CD-4 receptor, including all HIV.

sCD-4 proteins may be used in combination with other agents, for example, in association with agents directed against other HIV proteins, such as reverse transcriptase, protease or tat. An effective therapeutic agent against HIV should prevent virus mediated as well as cell to cell transmission of infection. sCD-4 proteins may also be used in combination with other anti-viral agents, for example, azidothymidine (AZT).

The sCD-4 proteins of this invention also have utility as inhibitors of CD-4[+] cell function by binding to extracellular target molecules which normally interact with the surface domain of the CD-4 receptor. Numerous studies suggest a critical role for the CD4 receptor in immune tolerance, particularly in the pathogenesis and progression of autoimmune disease and in host specific graft rejection.

As a prophylactic or therapeutic, sCD-4 proteins are administered parenterally, preferably intravenously. The agent can be administered by infusion or by injection, e.g., daily, weekly or monthly. The amount and rate of administration is selected so as to maintain an effective amount of sCD-4 protein in circulation. An alternative mode of administration would be extracorporeal, employing sCD-4 proteins as dialysis agents.

The sCD-4 protein of this invention can also be used as a reagent to identify natural, synthetic or recombinant molecules which act as therapeutic agents or inhibitors of CD-4[+] cell interactions.

For example, sCD-4 proteins can be used in screening assays, such as, assays for protein interaction measured by ELISA based methodologies, to provide a biochemically pure, aqueous soluble reagent which may be used in combination with other reagents to assay for competitors of the CD-4 receptor surface domain interactions. For example, since sCD-4 proteins bind to HIV env protein or mixtures containing HIV env proteins, the proteins can be used to screen for inhibitors of virus binding.

Based on in vitro data showing that sCD-4 proteins bind to cells expressing HIV env proteins, these proteins can also serve as selective targeting molecules for HIV infected cells in vivo. As a target specific carrier protein, sCD-4 proteins can serve, for example, as the carrier protein for delivery of cytotoxic agents to the infected cells.

In addition, based on data showing that the CD-4 receptor specifically associates with MHC class II antigens on antigen presenting cells as suggested by the class restriction of CD-4[+] cells, sCD-4 proteins can be used in combination with class II antigens to test for inhibitors of CD-4 lymphocyte - target cell interactions. In addition to these examples, which are based on direct binding assays between the protein and its target molecules, more complex assays can be designed which rely on the biochemical responses to protein recognition. The deletion mutants can be used in diagnostic assays for the detection of CD-4 proteins or the molecules with which they interact. For example, quantitation of CD-4 and CD-4$^+$ cells and antibodies to CD-4 would be of diagnostic value for AIDS.

In addition, sCD-4 proteins can be used to generate new diagnostic reagents, for example, Mabs or other types of molecules for use in the standard immunologic assays, i.e., ELISA, capture immunoassays, radioimmune assays. Because the sCD-4 display the OKT-4, the OKT-4A and most if not all of the other surface epitopes of the CD-4 receptor, the proteins are especially useful in immunodiagnostic assays for absolute quantitation of CD-4 levels in a system.

The CD-4 receptor resides in three diverse chemical environments: the oxidizing, hydrophilic cell surface; the hydrophobic membrane; and the reducing, hydrophilic cytoplasm. These diverse environments would most likely preclude the isolation of the receptor in its fully native state. The sCD-4 proteins consist of only select segments of the extracellular domain, are secreted as a soluble proteins into the cell supernatant and their conformation appears to mimic to a significant extent the surface of the receptor surface domain. Thus, the sCD-4 proteins are suitable for detailed structural analysis, in particular for x-ray crystallography. Determination of the 3-dimensional structure of the sCD-4 proteins alone or in a complexed form with other interactive molecules could provide a basis for the rational design of selective antagonists and agonists for sCD-4.

## Examples

The Examples which follow are illustrative, and not limiting of the invention. Enzymes used in the genetic manipulations were obtained from commercial sources and were used substantially in accordance with the vendor's instructions. Except where otherwise indicated, procedures were carried out substantially as described by Maniatis, et al., Molecular Cloning, Cold Spring Harbor Laboratory, 1982.

## Example 1:

### Construction of E. coli expression vectors

Using recombinant DNA manipulations, the coding sequence for the full length SKBsCD-4 and the deletion mutants, V1V2J4 or V1J4, followed by an in frame TAA termination codon (see sequence, above) were placed downstream of the OMPA signal sequence in an E. coli expression vector to create plasmids OMPAST4BBV1, OMPAV1V2 and OMPAV1. The construction of these vectors is as follows:

### Construction of plasmid JRT4:

To create plasmid JRT4, plasmid DSP1 (Pfarr, et al., DNA 4:461(1985)) was cut with XhoI, the SV40 polyA early region was deleted, and the XhoI sites were filled in using Klenow fragment of DNA polymerase. The bovine growth hormone (BGH) polyadenylation region (Pfarr, et al., DNA 5:115(1986)) was cut with PvuII and KpnI, and the KpnI site was blunted by treatment with T4 DNA polymerase. This 230 bp fragment was ligated to DSP1 to create DSP1BGH.

DSP1BGH was cut with SmaI and SalI and the galK cassette (consisting of the SV40 early promoter, galK coding region and BGH polyA region) was ligated into pUC19 (Yanisch-Perron, et al., Gene 33:103-(1985)) at the SalI site by using a synthetic linker consisting of a SalI end, a BglII site, and a SmaI end. This three part ligation resulted in plasmid DSP1BZBGH.JT.

DSP1BZBGH.JT, which was cut with StuI and BclI to delete the galK coding region, was ligated to a 1.7 kb EcoR1 (filled in)-BamHI fragment, containing the CD-4 cDNA from plasmid pT4B (Maddon, et al., Cell 42:93(1985)) to create plasmid JRT4.

### Construction of plasmid pUCsT4:

To create plasmid pUCsT4, a HaeII and HpaII fragment (1125 bp) of the CD-4 cDNA from plasmid pT4b was ligated through the use of synthetic linkers, to vector pUC18 which had been cut KpnI and XbaI. The HaeII end of the CD-4 cDNA was ligated to the KpnI site of pUC18 using a synthetic linker with a KpnI end and a HaeII end. The HpaII end of the CD-4 cDNA was ligated to the XbaI site of pUC18 using a synthetic linker with a HpaII end and an XbaI end. This linker also inserted a TAA stop codon after nucleotide 1257 of the CD-4 coding region. The resulting plasmid was pUCsT4.

Construction of pST4sal:

To create plasmid pST4sal, plasmid JRT4 was cut with BglII and SacI and a 959 bp fragment (consisting of the SV40 early promoter and the first 602 nucleotides of the CD-4 cDNA) was isolated. Plasmid pUCsT4 was cut with SacI and XbaI and a 660 bp fragment (consisting of the CD-4 cDNA from nucleotides 603-1257 followed by the TAA codon from the synthetic linker) was isolated. These two fragments were ligated into DSP1BZBGH.JT which had been cut with BglII and XbaI to delete the SV40 early promoter and full length CD-4 coding region. The resulting plasmid was pST4sal.

Construction of pST4DHFR:

To create plasmid pST4DHFR, a BglII-BamHI fragment containing a β-globin DHFR expression cassette was ligated into the BamH1 site of pST4sal. The β-globin DHFR expression cassette consists of: the mouse β-globin promoter (550 bp HincII fragment from plasmid pPk288 (Berg, et al., Mol. Cell. Biol. 3:1246(1983)) modified at its 5′ end with a synthetic linker to contain a BglII site; the mouse DHFR coding region (735 bp HindIII (fill-in) fragment from plasmid pSV2-DHFR (Subramani, et al., Mol. Cell. Biol. 1:854(1981)); NheI (fill-in)-BamHI (fill-in) SV40 polyA early region from DSP1 (Pfarr, et al., DNA 4:461(1985)); and the mouse DHFR terminator region (907 bp HindIII (fill-in fragment from plasmid mDH9 (Frayne, et al., Mol Cell. Biol. 4:2921-(1984)), modified at its 3′ end with a synthetic linker to create a BamHI site.

Construction of psT4BBV1DHFR:

To create plasmid psT4BBV1DHFR, plasmid pST4DHFR was cut with EcoRI and XbaI and the smaller fragment containing the sCD-4 coding region was deleted. Plasmid sT4sal was cut with XbaI and BbvI and a 1120 base pair coding fragment containing the sequence for soluble CD-4 minus the leader region was isolated. This fragment was ligated to the EcoRI/XbaI-cut pST4DHFR, using a synthetic linker with an EcoRI end, a KpnI site, and a BbvI end. This fragment is compatible with the BbvI overhang on the sCD-4 fragment isolated above. The resulting plasmid was called pST4BBV1DHFR.

Construction of OMPAST4:

To create plasmid OMPAST4, plasmid OMPA.GS was digested with NcoI and SalI and the smaller fragment containing the linker region was deleted. OMPA.GS is a derivative of pAS1 (Rosenberg et al., Meth. Enzymol. 101:123 (1983); U.S. Patent 4,578,355) having a synthetic sequence inserted into the NdeI site at the 3′ end of the cII ribosome binding sequence. The synthetic sequence comprises the OMPA leader followed by a multilinker sequence. The synthetic sequence was substantially as follows:

```
5'-T ATG AAA AAG ACA GCT ATC GCG ATT GCA GTG GCA CTG GCT

GGT TTC GCT ACC GTA GCG CAG GCC GGC TCT AGA GTC GAC CTA

GTT AAC TAG-3'
```

Plasmid pUCsT4 was cut with NcoI and SalI and the 1149 base pair fragment containing sCD-4 sequence (CD-4 nucleotides 124-1257) was isolated. This fragment was ligated to the NcoI/SalI cut OMPA.GS to create OMPAST4.

### Construction of OMPAST4Bbvl:

To create plasmid OMPAST4Bbvl, plasmid OMPAST4 was cut with Nael and Xbal. The smaller fragment resulting from this cut, containing the sCD-4 coding region was deleted. Plasmid ST4BBVIDHFR was cut with Kpnl and the resulting 3' overhang was blunt ended with T4 DNA polymerase. The blunt ended DNA was then cut with Xbal and the 1124 base pair fragment containing the nucleotides 145-1257 of the CD4 cDNA was isolated. The isolated fragment was ligated to the Nael/Xbal cut OMPAST4 plasmid to create plasmid OMPAST4Bbvl.

### Construction of pucST4184:

To create plasmid pucST4184, an EcoRI-Nhel fragment from the CD-4 cDNA [a 682 bp fragment encoding amino acids (-25) to (+176)] was ligated to the synthetic linker sk727/725 (Nhel and Aval ends) at the Nhel site. sk727/725 codes for CD-4 amino acids 177-183). The Aval end of sk727/725 was ligated to an Aval-Xba 1 fragment of pucST4 (comprising bp 1198-1257 of the human cDNA and encoding amino acids 351-369 of the CD-4 receptor followed by a TAA termination codon). This sequence, which is flanked by EcoRI and Xbal ends, was inserted into pUC19 at the EcoRI and Xba 1 ends in the pUC19 polylinker (sk727 725). sk727 725 is substantially as follows:

$$5' \ \text{ctagctttccagaaggcc} \ 3'$$
$$3' \ \text{gaaaggtcttccggagcc} \ 5'$$

### Construction of pucST4106:

To create plasmid pucST4106, an EcoRI-Ava II fragment (comprising bp 1-413, and encoding amino acids (-)25 to 87) of the CD-4 cDNA was joined to the synthetic linker sk791/792 (Ava II-Aval ends) at the Avall site. sk791-792 codes for CD-4 amino acids 88-104. The Aval end of sk791/792 was ligated to an Aval-Xbal fragment of pucST4 (comprising bp 1198-1257 of the human cDNA, and encoding amino acids 351-369 of the CD-4 receptor followed by a TAA termination codon). This sequence, which is flanked by EcoRI and Xbal, ends was inserted into pUC19 at the EcoRI and Xbal ends in the pUC19 polylinker. sk791 792 is substantially as follows:

$$5' \ \text{gaccagaaggaggaggtgcaattgctagtgttcggattgactgccaac}$$
$$\text{gtcttcctcctccacgttaacgatcacaagcctaactgacggttgagc} \ 5'$$

### Construction of sT4184.DHFR:

To create plasmid sT4184.DHFR, an EcoRI-Xbal fragment of pucST4184 (encoding amino acids -25 to 183 fused to 351 to 369) was ligated to the EcoRI and Xbal ends of psT4.DHFR in place of the EcoRI-Xbal fragment encoding SKBsCD-4.

### Construction of sT4106.DHFR:

To create plasmid sT4106.DHFR an EcoRI-Xbal fragment of pucST4106 (encoding amino acids -25 to 104 fused to 351 to 369) was ligated to the EcoRI and Xbal ends of psT4.DHFR in place of the EcoRI-Xba 1 fragment encoding SKBsCD-4.

### Construction of OMPAV1:

12

To create OMPAV1, plasmid OMPAST4Bbvl was cut with AflII and XbaI and the smaller fragment containing nucleotides 371-1257 of the CD-4 cDNA coding region was deleted. Plasmid pst4106.DHFR was cut with AflII and XbaI and a 160 base pair fragment containing nucleotides 371-459, 1198-1257 of the CD-4 cDNA sequence was isolated. The two fragments were ligated to create plasmid OMPAV1.

## Construction of OMPAV1V2:

To create OMPAV1V2, plasmid OMPAST4Bbvl was cut with SacI and XbaI and the smaller fragment containing nucleotides 603-1257 of the CD-4 cDNA sequence was deleted. Plasmid ST4184.DHFR was cut with SacI and XbaI and a 164 base pair fragment containing nucleotides 603-696, 1198-1257 of the CD-4 cDNA sequence was isolated. The two fragments were ligated to create plasmid OMPAV1V2.

## Example 2:

### Expression of sCD-4 proteins in E.coli

The OMPAV1 and OMPAV1V2 constructions were transformed into defective E. Coli lambda lysogens, AR58 (cl857) and AR120 using standard procedures. Heat inductions (Rosenberg, et al., Meth. Enzymol. 101:123 (1983)) or naladixic acid inductions (Mott, et al., Proc. Natl. Acad. Sci. USA 82:88 (1985)) were performed as described below.

### Heat Induction:

For cell line AR58, 200 ml of LB (containing 50 ug/ml ampicillin) was seeded with 7 ml from a 10 ml overnight (O.N.) culture of AR58 cells containing the OMPAST4Bbv1, OMPAV1 or OMPAV1V2 plasmid, and grown in a shaking incubator at 32° C. At an optical density at 650 nm (O.D.$_{650}$) of 0.8 absorbance units, 200 ml of LB, warmed to 55° C, was added to the culture and the flask was transferred to a 42° C shaking incubator. One ml samples of the culture were taken 30, 60 and 90 minutes after the temperature shift, for analysis. At 90 minutes, the cells were chilled for 15 minutes in ice water, and then pelleted at 3500 rpm for 15 minutes at 4° C in a J-6 Beckman centrifuge (Beckman Instruments, Fullerton, California). Cell pellets were stored frozen at -20° C until processing. Conditioned media was stored frozen at -20° C until processing.

### Nalidixic Acid Induction:

For cell line AR120, 400 ml of LB (containing 50 ug/ml ampicillin) was seeded with 13 ml from a 20 ml O.N. culture of AR120 cells containing the OMPAST4Bbv1, OMPAV1 or OMPAV1V2 plasmid, and grown in a shaking incubator at 37° C. At an C.D.$_{650}$ of 0.4, 400 ul of nalidixic acid (50 mg/ml) were added to the culture. The culture was maintained at 37° C in a shaking incubator and 1 ml samples were taken 1, 3 and 5 hours after addition of the nalidixic acid. At 5 hours, the cells were chilled for 15 minutes in ice water, and then pelleted at 3500 rpm for 15 minutes at 4° C in a J-6 Beckman centrifuge. Cell pellets were stored frozen at -20° C until processing. Conditioned media was stored frozen at -20° C until processing.

Following induction, cells were pelleted from the 1 ml samples, and analyzed for expression of the full length and mutant sT4 proteins (SKBsCD-4, V1J4 and V1V2J4) by western blot, using a rabbit polyclonal antibody to a denatured form of soluble T4 produced in bacteria. Proteins of the appropriate sizes were detected in cell lysates of both heat and nalidixic induced samples. Levels detected represented 1-5% of total protein.

### Conditioned Media Processing:

Frozen conditioned media was thawed at room temperature and 30 ml aliquots were taken and centrifuged at 4° C for 1 hour at 24,000 rpm in a Beckman SW28 swinging bucket rotor. Following centrifugation, the supernatants were pooled and concentrated 10-20 fold at 4° C by pressure membrane filtration.

Concentrated media was assayed for full length SKBsCD-4, V1J4, or V1V2J4 proteins by western blot analysis (as above), competition ELISA, and immunoprecipitation.

In each sample, protein of the appropriate size was detected in the concentrated conditioned media by western blot, at levels representing 1-5% (0.1-0.5 ng/ml unconcentrated conditioned media) of that detected inside the cell. All 3 proteins bound OKT4A in the competition ELISA assay. The V1J4 and V1V2J4 proteins were further assayed by immunoprecipitation with a panel of monoclonal antibodies to CD-4 (Ortho, Raritan, New Jersey). The protein expressed by OMPAV1, V1J4, was immunoprecipitated by OKT4A, and OKT4D but not by OKT4, OKT4B, OKT4C, OKT4E or OKT4F. The protein expressed by OMPAV1V2, V1V2J4, was immunoprecipitated by all of the OKT4 monoclonal antibodies except OKT4. In addition, the affinity of this protein for OKT4C was lower than for the other monoclonals.

Cell Pellet Processing:

Cell pellets from the 90 minute time point of the OMPAST4Bbv1/AR58 heat induction, were thawed on ice, and resuspended in buffer (100mM Tris-HCl, pH 8, 0.5 mM EDTA, and 0.5 M sucrose) at 4° C at a pellet to buffer raio of 100 O.D._{650}/ml. Lysozyme (10 mg/ml in 0.05 M Tris-HCl, pH 8) was added to a final concentration of 0.2 mg/ml and the suspension incubated on ice for 15 minutes. An equal volume of ice cold water was added and the suspension incubated on ice for 15 minutes. PMSF (50 mM) was added to a final concentration of 1 mM, followed by the addition of MgCl₂ (1 M) to a final concentration of 2 mM. The cell suspension was passed through an 18 gauge needle 3 times to reduce viscosity, and then centrifuged at 20,000 x g for 10 minutes, at 4° C.

Following centrifugation, the pellet was resuspended in 20 mM Tris-HCl, pH 8, 5 mM EDTA, 5 mM βME, in a volume equivalent to that prior to centrifugation. The suspension was sonicated for 60 seconds (10 second bursts with 30 seconds in between for cooling), and then centrifuged at 20,000 x g for 15 minutes at 4° C. Western blot assay of the resulting pellet and supernatant, indicated that 70% of the SKBsCD-4 protein was in the supernatant fraction. The supernatant fraction was centrifuged at 100,000 x g for 60 minutes. Western blot analysis of the resulting pellet and supernatant, indicated that <5% of the SKBsCD-4 protein had remained in solution. The supernatant fraction was diluted with an equal volume of 40 mM MES pH 6.0 and loaded onto a 1 ml S Sepharose (Pharmacia, Piscataway, New Jersey) column equilibrated with 20 mM MES pH 6.0. The column was washed with 10 mls 20 mM MES pH 6.0. The bound SKBsCD-4 was eluted with 20 mM MES pH 6.0, 0.5 M NaCl, collecting 1 ml fractions. Western blot analysis of the flow through, wash, and elution fractions, indicated that <5% of the SKBsCD-4 bound to the column and eluted in fractions 2 and 3.

The flow through, wash and elution fractions were assayed for functional SKBsCD-4 by competition ELISA. Both the flow through and elution fraction 2 contained SKBsCD-4 which bound OKT4A.

Immunoprecipitation:

Concentrated media (100 ul) was diluted with an equal volume of precipitation buffer (10 mM sodium phosphate, pH 7.5, 100mM NaCl, 0.1% NP-40, 0.5% non-fat dry milk) and precleared with 3 ug rabbit IgG for 15 minutes at 4 C, followed by the addition of 30 ul (packed volume) of protein A-Sepharose beads (Pharmacia, Piscataway, New Jersey) for 30 minutes at 4° C. Precleared supernatants were incubated with 5 ug of OKT4, 4A, 4B, 4C, 4D, 4E, 4F, OKT8 monoclonal antibodies (Ortho Pharmaceuticals), mouse IgG (Cooper Biomedical, Malvern, Pennsylvania) or rabbit anti-mouse IgG (Cooper Biomedical) for 30 minutes at 4 C. OKT4, 4A, 4C, OKT8, mouse IgG and rabbit anti-mouse IgG were precipitated by incubation with 20 ul (packed volume) of protein A-Sepharose beads for 30 minutes at 4° C. OKT4B, 4D, 4E and 4F were incubated with 10 ug of rabbit anti-mouse IgG for 30 minutes at 4° C prior to addition of the protein A Sepharose beads. Following precipitation, the beads were washed twice with 200 ul of precipitation buffer and once with 200 ul of precipitation buffer without NP-40 and non-fat dry milk. The washed beads were boiled for 5 minutes in 20 ul of sample buffer (125 mM Tris-HCl, pH 6.8, 20% glycerol, 1.4M βME (beta-mercaptoethanol)). Following boiling, the supernatants were electrophoresed on a 12.5% SDS-polyacrylamide gel and analyzed by western blot as described above.

Both V1J4 and V1V2J4 proteins were immunoprecipitated by OKT4A. The protein expressed by OMPAV1 was immunoprecipitated by OKT4A and OKT4D, but not by OKT4, OKT4B, OKT4C, OKT4E or OKT4F. The protein expressed by OMPAV1V2 was immunoprecipitated by all of the OKT4 monoclonal antibodies except OKT4. In addition, the affinity of this protein for OKT4C was lower than for the other monoclonal antibodies.

Competition ELISA:

Microtiter plates (Flow Laboratories, McLean, Virginia) were coated overnight at room temperature with 100 ul/well of 0.1 M NaHCO3/Na2CO3 (pH 9.4) containing 150 ng of purified sCD-4.

Aliquots (2.5-80 ul) of concentrated media or cell extract were incubated with 5 ng of OKT4A in the presence of 1 mg/ml bovine serum albumin (BSA) in a final volume of 100 ul, overnight at room temperature. Samples of less than 100 ul were diluted to 100 ul with phosphate buffered saline (PBS). After incubation, the plates were poured off, rinsed 3 times with PBS and patted dry. To each well was added 380 ul of PBS/0.5% gelatin. The plates were incubated at 37° C for one hour.

Following incubation at 37° C, the plates were poured off, rinsed 3 times with PBS and patted dry. The 100 ul sample incubations containing the monoclonal antibody were added to the individual wells of the prepared plate and incubated for 5 hours at 37° C.

Following incubation, the plates were poured off, rinsed 5 times with PBS and patted dry. 100 ul of a solution containing biotinylated horse/anti-mouse IgG was added to each well. This antibody preparation was prepared by adding one drop of horse/anti-mouse IgG (Vector Labs, Burlingame, California) to 10 ml of PBS containing 1% horse serum. The plates were incubated for 30 minutes at 37° C.

Following incubation, the plates were poured off, rinsed 5 times with PBS and patted dry. 100 ul of ABC complex (Vector Labs, Burlingame, California) was added to each well. The plates were then incubated at room temperature for 30 minutes.

The plates were again poured off, rinsed 5 times with PBS and patted dry. 100 ul of p-nitrophenyl phosphate mix (2 mg/ml p-nitrophenyl phosphate (Sigma Chemical Co., St. Louis, Missouri) in 0.1 M Sodium bicarbonate, pH 9.4, 10 mM MgCl2) was added to each well. The plates were incubated at 37 C in the dark for 30 minutes. Following the incubation, the plates were read at an absorbance of 405 nm. Concentration of sCD-4 were determined against a standard curve generated by incubating known amounts of purified sCD-4 with the OKT4A antibody. Both proteins (V1J4 and V1V2J4) were found to bind OKT4A monoclonal antibody in this competition ELISA.

Example 3:

Construction of yeast expression vectors

The plasmids used to construct expression vectors in yeast for the expression of sCD-4 proteins are based on the basic high copy yeast shuttle vector pYSK102 (Rosenberg, et al., Genetic Engineering 8:151- (1986). All such plasmids contain an 823 base pair EcoRI-PstI fragment of the yeast TRP1 gene (Tsumper and Carbon, Gene 10:157 (1980)), which provides a selectable marker; a 2.0 kb EcoRI-PstI fragment of the yeast 2-micron circle (Hartley and Donaldson, Nature 286:860(1980)), which provides an origin of replication in yeast; a portion of pBR322 (deleted from BamHI to PvuII) (in Cloning Vectors, Pouwels, Enger, Valk, Eds., 1985), which provides for replication and selection in E.coli and a yeast promoter-terminator module inserted between the HindIII and BamHI sites of pBR322. The promoter is either a 431 base pair BamHI-RsaI fragment of CUP1 derived as a BamHI-EcoRI fragment from pYSK12 (Butt, et al., Proc. Natl. Acad. Sci. USA 81:3332 (1984); Rosenberg et al., Genetic Engineering 8:151 (1986); Gorman, et al., Gene 48:13 (1986)) or a 1.1 kb TDH3 promoter sequence (Holland, et al., J. Biol. Chem. 255:2596(1980)) derived as a BamHI-EcoRI fragment from plasmid pYSK18 (Rosenberg, et al., supra; Gorman, et al., supra). The terminator is a 361 base pair KpnI-HindIII fragment of the CYC1 gene (Smith, et al., Cell 16:753 (1979)). Into the region between the promoter and the CYC1 fragment can be inserted various synthetic sequences as described below.

Construction of pYSK147 and pYSK148:

Plasmids pYSK137 (CUPI promoter) and pYSK138 (TDH3 promoter) contain a synthetic oligonucleotide sequence between the EcoRI site at the 3' end of the promoter sequence and a KpnI site at the 5' end of the terminator sequence. This synthetic sequence contains the unique restriction sites XhoI, NcoI and SalI upstream of translational termination codons in all three reading frames. The synthetic linker has the sequence:

GAATTCTCGAGCCATGGCCAGTCGACGTAGTTAAGTAGGTACC

A NcoI-SalI fragment of plasmid pUCsT4, containing bases 124 through 1257 of the sCD-4 sequence was inserted into NcoI-KpnI cut pYSK137 or pYSK138 to yield pYSK147 and pYSK148 respectively.

## Construction of pYSK154 and pYSK155:

Vectors for ubiquitin fusion gene expression contain a cassette adapted synthetic yeast ubiquitin gene (Ecker, et al., J. Biol. Chem. 262:3524(1987)) between the promoter and the CYC1 terminator. The ubiquitin sequence is identical to that published by Ecker, et al., except that the terminal AflII-KpnI fragment was replaced by an oligonucleotide sequence containing an NcoI restriction site at the 3' end. The NcoI-KpnI fragment of pYSK147, containing the desired sCD-4 sequence was inserted between this NcoI site and the KpnI site at the 5' end of the CYC1 fragment, to give pYSK154. A derivative of pYSK154, in which the sequence for the first seven amino-terminal amino acids of the sCD-4 sequence in pYSK154 (the leader peptide sequences) were deleted, was constructed by cleavage of pYSK154 with KpnI, followed by fill-in with the Klenow fragment of E.coli DNA polymeraseI (polIK) and insertion of a KpnI-XbaI sCD-4 sequence (bases 145 through 1257 of sCD-4) from psT4BBV1DHFR which had been treated with polIK to fill-in the sticky ends. This plasmid is designated pYSK155.

## Construction of pYSK159 and pYSK161:

The vectors containing a truncated sCD-4 sequence (bases 124-603) were constructed by inserting a synthetic oligonucleotide sequence containing a termination codon at the SacI site of the sCD-4 sequence (base 602). The synthetic linker (2x stranded) has the sequence:

$$\text{CTAAGCGGC}$$
$$\text{TCGAGATTCGCCGGC}$$

This synthetic sequence was inserted into SacI and NarI cut pYSK148 and pYSK154 to give pYSK159 and pYSK161 respectively. Thus, the CD-4 DNA in pYSK159 has the codons for amino acids -9 (alanine) through 151 (leucine)(YV1).

## Example 4:

### Expression of sT4 in yeast cultures.

Plasmid DNA (2-10 ug/ml) was used to transform yeast (Saccharomyces cerevisiae) using standard techniques (Sherman et al, Methods in Yeast Genetics, Cold Spring Harbor, 1986), selecting for tryptophan prototrophy. The strains employed were strain F762 (Mata, trp1Δ1, ura3-52) or PEC1-8B (Mata,proAΔ: : URA3.ura3-52, trp1-289, his3). Cultures of selected transformants were grown in Yeast Nitrogen Base (YNB, Difco, Detroit, Michigan) containing supplements (2% glucose, 20 ug/ml uracil, 20 ug/ml histidine). For plasmids utilizing the CUP1 promoter, copper sulfate at 100 micromolar was included. Cultures were harvested in late log phase by centrifugation and were washed and broken. The cell lysate was analysed for sCD-4 by standard protein immunoblot assay.

In S. cerevisiae containing pYSK147 and pYSK148, the sCD-4 protein, SKBYsCD-4, (amino acids -9 to 369) was expressed at low levels (less than about 0.5 ng/l). The activity was found to be associated with the particulate fraction, but could be readily solubilized by extraction with non-ionic detergents. The amount of SKBYsCD-4 (amino acids -9 to 369) in cultures of S. cerevisiae containing pYSK154 was between about 2 to about 6 mg/liter (at $2\times10^8$ cells/ml). This was about a five-fold improvement over the amount of the CD-4 protein expressed with pYSK147 and pYSK148, i.e., without the presence of the stabilizing ubiquitin sequence. The estimated size of the sCD-4 protein synthesized was identical in all cases, indicating that the ubiquitin portion of the protein had been cleaved from SKBsCD-4. This was confirmed by immunoblotting procedures, using an antibody to yeast ubiquitin.

The levels of expression of the truncated sCD-4 proteins (YV1) in pYSK159 and pYSK161 were similar to that of the sCD-4 protein expressed as a ubiquitin fusion. The amount of sCD-4 detected was similar in each of the host strains examined.

SKBsYCD-4 from strain F762 containing pYSK154 was partially purified by passage through an S-Sepharose column. A crude extract, prepared in 10 mM Tris buffer, pH 6.0, 1% Tween-20, was centrifuged at 30,000xg. The supernatant was adjusted to pH 4.0 and was applied to an S-Sepharose column equilibrated in 50 mM acetate buffer. The sample was batch eluted with 50 mM MES (2-(N-morpholino)-ethanesulfonic acid], pH 6.0) buffer containing 0.5M NaCl. The presence of SKBYsCD-4 in the eluate was confirmed by immunoblot assay. The material was active as determined by a competition ELISA based assay, utilizing purified SKBsCD-4 from CHO cells and OKT4A antibody, suggesting that the conformation of the yeast derived SKBsYCD-4 mimics that of the mammalian cell (CHO) derived material.

Example 5:

Construction of Streptomyces vectors

Using recombinant DNA manipulations, segments of the human CD-4 cDNA sequence were fused to the signal sequence of the S. longisporus trypsin inhibitor (LTI) gene and the S. lividans β-galactosidase (βgal) gene. The sCD-4 minigenes were joined to Streptomyces plasmids pIJ702 (Katz, et al., supra) and pIJ351 (Kieser, et al., supra) to create plasmids pLTI:sT4/1, pLTI:sT4/7, pLTI:V1V2 and pBgal:sT4/7. The construction of the vectors was as follows:

Construction of pLTI:sT4/1:

Plasmid pLTI:sT4/1 was constructed from three other plasmids: pUCsT4, pIJ351 and pLTI450. The construction of plasmid pLTI450 is as follows:

Construction of pLTI450:

A 0.92 kb SacI-KpnI fragment containing the LTI gene was inserted into pUC18 which had been digested with SacI and KpnI. This plasmid, pLTI520, was partially digested with EagI and totally digested with SalI then ligated to a synthetic linker (2x stranded) which had EagI and SalI ends:

$$5'-GGCCGCCGCCCCCGCG$$

$$CGGCGGGGGCGCAGCT-5'$$

Plasmids obtained from this ligation were screened for insertion of the synthetic linker into the EagI site located approximately 0.5 kb from the SacI site. This EagI site is located at base pair 86 with respect to the 5' end of the LTI gene. The resulting plasmid contains the LTI promoter and the coding sequence for the signal peptide and signal peptide cleavage site. Both the EagI and SalI sites can be used to fuse sCD-4 minigenes to the LTI signal sequence.

Construction of pLTI:sT4/1:

To create plasmid pLTI:sT4/1, the sCD-4 coding sequence was isolated on an approximately 1.1 kb BbvI-PstI fragment from pUCsT4. This fragment contains base pairs 149-1257 of the human CD-4 cDNA sequence. Plasmid pUCsT4 was digested with BbvI followed by treatment with reverse transcriptase to fill in the 5' protruding ends. The DNA was then digested with PstI. This 1.1 kb BbvI(RT)-PstI fragment, which encodes SKBsCD-4, was ligated to pLTI450 which had been treated with SalI and reverse transcriptase followed by PstI digestion. This ligation resulted in plasmid pLTI450sT4/1. The final step in the construction of pLTI:sT4/1 required the insertion of Streptomyces replication functions onto pLTI450sT4/1. This was accomplished by digesting pIJ351 (Kieser, et al., supra) with PstI and ligating it to PstI digested pLTI450sT4/1.

Construction of pLTI:sT4/7:

Plasmid pLTI:sT4/7 was constructed from three other plasmids: pUCsT4, pIJ702 and pLTI450. To create plasmid pLTI:sT4/7, the sCD-4 coding sequence was isolated on an approximately 1.1 kb NcoI-PstI fragment from pUCsT4. This fragment contains base pairs 124-1257 of the human CD-4 cDNA sequence. These base pairs were placed between an ATG initiation codon and a TAA termination codon as previously described in Example 1. The initiation codon makes up part of the NcoI recognition sequence. The sCD-4 coding sequence contained on this fragment contains 9 amino acids from the leader sequence of CD-4. pUCsT4 was treated with NcoI and reverse transcriptase followed by digestion with PstI. This fragment was then ligated with pLTI450 which had been treated with HincII and PstI. This ligation resulted in plasmid pLTI450sT4/7. The Streptomyces replicon was provided by plasmid pIJ702 (Katz, et al., supra). It was inserted into pLTI450sT4/7 via the unique PstI sites on both plasmids to create plasmid pLTI:sT4/7.

Construction of plasmid pLTI:V1V2:

Plasmid pLTI:V1V2 was constructed from plasmids psT4.184, pLTI450 and pIJ351. A 0.54 kb XbaI-EcoRI fragment was isolated from psT4.184. This fragment was treated with BbvI and reverse transcriptase. The BbvI end corresponds to base pair 149 of the human CD-4 cDNA sequence. This blunt-ended fragment was then ligated to pLTI450 which had been treated with SalI and reverse transcriptase to create plasmid pLTI450V1V2. The Streptomyces replication functions were provided by pIJ351. This plasmid was cloned into pLTI450V1V2 via the unique PstI site on both pIJ351 and pLTI450V1V2. The resultant plasmid was pLTI:V1V2.

Construction of pβgalsT4/7:

Plasmid pβgalsT4/7 was constructed from plasmids pUCsT4, pIJ702 and p3SSXMCP. p3SSXMCP is a pUC9 (Vieira and Messing, Gene 19:259(1982) derivative which contains the β-galactosidase promoter and signal sequence. Twenty-six base pairs downstream of the coding sequence for the signal peptide cleavage site is an XmnI site (Eckhardt, et al., J. Bacteriol. 169:4249(1987)). Inserted into this site was a synthetic linker which contains a BamHI recognition sequence (New England Biolabs, Beverly, Massachusetts) to generate plasmid p3SSX10. This vector was treated with BamHI and reverse transcriptase followed by XhoI digestion. Ligated to this vector was a fragment which had an NcoI end treated with reverse transcriptase to generate a blunt end and a SalI end. Ligation of the filled-in BamHI site with the filled-in NcoI site recreated both the BamHI and NcoI sites. The resulting plasmid was p3SSXMCP. pUCsT4 was treated with XbaI and reverse transcriptase followed by NcoI digestion. This 1.1 kb NcoI-XbaI(RT) fragment was then inserted into p3SSXMCP which had been treated with SacI and T4 DNA polymerase I followed by NcoI digestion. The Streptomyces replication functions were provided by pIJ702 which was inserted into p3SSXsT4 via the unique BglII site on both plasmids. The resulting plasmid was pβgalsT4/7.

Example 6:

Expression of sCD-4 minigenes in Streptomyces:

Plasmids pLTI:sT4/1, pLTI:sT4/7, pLTI:V1V2 and pβgal:sT4/7 were transformed into S. lividans 1326 (Bibb, et al., supra). In addition, pLTI:sT4/1, pLTI:sT4/7, pLTI:V1V2 were introduced into S. coelicolor M124 (Hopwood, et al., Genetic Manipulation of Streptomyces - A Laboratory Manual, F. Crowe & Sons, Ltd., Norwich, England (1985)), S. albus J1074 (Chater and Wilde, J. Gen. Microbiol. 116:323(1980)) and a white derivative of S. longisporus (ATCC accession number 23931). All of the plasmids were introduced into the various hosts using the procedure previously described by Thompson, et al., J. Bacteriol. 151:668(1982). Transformers were selected by overlaying the transformation plates with 3ml of 0.4% agar containing 100 μg/ml thiostrepton.

The clones which expressed CD-4 minigenes were identified by propagating thiostrepton-resistant transformants in ten milliliters of trypticase soy broth plus 5 μg/ml thiostrepton for 48-72 hours. Culture supernatants and cell lysates were separated on a 12% polyacrylamide (30:0.8 acrylamide:bis) - 1% sodium dodecylsulfate gel then transferred to nitrocellulose (Towbin, et al., Proc. Natl. Acad. Sci. USA 76:4350(1979)). The nitrocellulose filter was probed using the rabbit sCD-4-antiserum as described by Deen, et al., supra.

The pLTI:sT4/1, pLTI:sT4/7 and pβgal:sT4 S. lividans transformants all expressed a protein of approximately 45kD which is the predicted size of the sCD-4 protein encoded by these plasmids. Similarly, the pLTI:V1V2 S. lividans transformants expressed a protein of approximately 20kD which is the predicted size of the V1V2 protein. SKBsCD-4 and V1V2J4 expression was also examined in S. coelicolor, S. albus, and S. longisporus. Like S. lividans, proteins of 45 kD (the pLTI:sT4/1 and pLTI:sT4/7 transformants) and 20 kD (the pLTI:V1V2 transformants) were expressed by the other Streptomyces species.

Subsequent quantitation of SKBsCD-4 present in the S. lividans culture supernatants and cell lysates showed levels of 1-5 mg/l. V1V2J4 was present at 2-10 mg/l in both culture supernatants and cell lysates. Less than 1 mg/l was expressed by the pβgal:sT4 S. lividans transformants.

Using a pLTI:sT4/7 S. lividans cell line, SKBsCD-4 expression has also been examined in a 12 liter fermentor. Similar to the small scale expression studies, cells were grown in trypticase soy broth plus 5 μg/ml thiostrepton. Both culture supernatants and cell lysates were analyzed for SKBsCD-4 expression at 7.5, 24, 31.5, 54 and 72 hours after inoculation. The maximum volumetric expression level was 60 mg/l and occurred at 24 hours after inoculation. The SKBsCD-4 was equally distributed between the culture supernatant and intact cells.

Partially purified supernatants containing V1V2J4 and SKBsCD-4 were shown by competition ELISA to bind OKT4A, indicating that these Streptomyces-derived proteins are HIV binding proteins.

The HIV binding protein, V1J4, was similarly ligated to the LTI promoter and shown by western assay to be expressed in S. lividans.

The above Examples demonstrate that HIV binding proteins derived from CD-4, e.g., SKBsCD-4, SKBYsCD-4, V1J4, V1V2J4 and YV1, which were expressed and secreted by the recombinant organisms of the invention are HIV binding proteins having the HIV binding function of sCD-4. More particularly, they have the HIV binding domain of the V1 region of SKBsCD-4.

The above description and Examples fully disclose the invention including preferred embodiments thereof. Modifications and improvements of the embodiments specifically disclosed herein are within the scope of the following claims.

## Claims

1. An E. coli expression vector comprising a coding sequence for a HIV binding protein operatively linked to a regulatory element.

2. The vector of claim 1 in which the HIV binding protein is a sCD-4 protein or a derivative thereof having the HIV binding function of a sCD-4 protein.

3. The vector of claim 1 in which the HIV binding protein is SKBsCD-4 or a derivative thereof having the HIV binding function of SKBsCD-4.

4. The vector of claim 3 in which the HIV binding protein is selected from the group consisting of SKBsCD-4, V1J4 and V1V2J4.

5. The vector of claim 1, 2, 3 or 4 in which the regulatory element comprises a promoter and a leader sequence.

6. The vector of claim 5 in which the leader sequence is a OMPA leader sequence.

7. An E. coli transformed with the vector of any of claims 1 through 6.

8. A Streptomyces expression vector comprising a coding sequence for a HIV binding protein operatively linked to a regulatory element.

9. The vector of claim 8 in which the HIV binding protein is a sCD-4 protein or a derivative thereof having the HIV binding function of a sCD-4 protein.

10. The vector of claim 8 in which the HIV binding protein is SKBsCD-4 or a derivative thereof having the HIV binding function of SKBsCD-4.

11. The vector of claim 10 in which the HIV binding protein is selected from the group consisting of SKBsCD-4, V1J4 and V1V2J4.

12. The vector of claim 8, 9, 10 or 11 in which the regulatory element comprises a promoter and a leader sequence.

13. The vector of claim 12 in which the regulatory element is the Longisporus Trypsin Inhibitor promoter and leader sequence or the Streptomyces beta-galactosidase promoter and leader sequence.

14. A Streptomyces transformed with the vector of any of claims 9 through 13.

15. The Streptomyces of claim 14 which is S. lividans, S. coelicolor, S. longisporus or S. albus.

16. A yeast expression vector comprising a coding sequence for a HIV binding protein operatively linked to a regulatory element.

17. The vector of claim 16 which is a S. cerevisiae vector in which the HIV binding protein is a sCD-4 protein or a derivative thereof having the HIV binding function of a sCD-4 protein.

18. The vector of claim 16 which is a S. cerevisiae vector in which the HIV binding protein is SKBsCD-4 or a derivative thereof having the HIV binding function of SKBsCD-4.

19. The vector of claim 18 in which the HIV binding protein is selected from the group consisting of SKBsYCD-4 and YV1.

20. The vector of claim 16, 17, 18 or 19 in which the regulatory element comprises a promoter and a leader sequence.

21. The vector of claim 16, 17, 18, or 19 in which the HIV binding protein coding sequence is fused at its 5' end to the 3' end of a coding sequence which stabilizes expression of the HIV binding protein.

22. The vector of claim 21 in which the coding sequence which stabilizes expression is the coding sequence for yeast ubiquitin.

23. The vector of claim 22 in which the regulatory element is the CUP 1 promoter or the TDH3 promoter.

24. A yeast transformed with the vector of any of claims 16 through 23.

25. A Saccharomyces cerevisiae transformed with the vector of claim 22 or 23.

26. A process for producing a HIV binding protein which comprises culturing either E. coli, Streptomyces or yeast cells, which have been transformed with a vector comprising a coding sequence for the HIV binding protein operatively linked to a regulatory element and collecting the HIV binding protein therefrom.

27. An HIV binding protein which is a sCD-4 protein or a derivative thereof having the HIV binding function of a sCD-4 protein and which is produced in recombinant E. coli, recombinant yeast cells or recombinant Streptomyces.

28. An E. coli expression vector for expressing exported proteins in E. coli which comprises a DNA coding sequence operatively linked to a regulatory element wherein the DNA coding sequence codes for the protein X-Y wherein X is an OMPA leader sequence and Y is a heterologous protein.


Claims for the following Contracting State: ES

1. A process for producing a HIV binding protein which comprises culturing E. coli cells which have been transformed with a vector comprising a coding sequence for the HIV binding protein operatively linked to a regulatory element and collecting the HIV binding protein therefrom.

2. The process of claim 1 in which the HIV binding protein is a sCD-4 protein or a derivative thereof having the HIV binding function of a sCD-4 protein.

3. The process of claim 1 in which the HIV binding protein is SKBsCD-4 or a derivative thereof having the HIV binding function of SKBsCD-4.

4. The process of claim 3 in which the HIV binding protein is selected from the group consisting of SKBsCD-4, V1J4 and V1V2J4.

5. The process of claim 1, 2, 3 or 4 in which the regulatory element comprises a promoter and a leader sequence.

6. The process of claim 5 in which the leader sequence is a OMPA leader sequence.

7. A process for producing a HIV binding protein which comprises culturing Streptomyces cells which have been transformed with a vector comprising a coding sequence for the HIV binding protein operatively linked to a regulatory element and collecting the HIV binding protein therefrom.

8. The process of claim 7 in which the HIV binding protein is a sCD-4 protein or a derivative thereof having the HIV binding function of a sCD-4 protein.

9. The process of claim 7 in which the HIV binding protein is SKBsCD-4 or a derivative thereof having the HIV binding function of SKBsCD-4.

10. The process of claim 9 in which the HIV binding protein is selected from the group consisting of SKBsCD-4, V1J4 and V1V2J4.

11. The process of claims 7, 8, 9 or 10 in which the regulatory element comprises a promoter and a leader sequence.

12. The process of claim 11 in which the regulatory element is the Longisporus Trypsin Inhibitor promoter and leader sequence or the Streptomyces beta-galactosidase promoter and leader sequence.

13. A process for producing a HIV binding protein which comprises culturing yeast cells which have been transformed with a vector comprising a coding sequence for the HIV binding protein operatively linked to a regulatory element and collecting the HIV binding protein therefrom.

14. The process of claim 13 in which the vector is a S. cerevisiae vector in which the HIV binding protein is a sCD-4 protein or a derivative thereof having the HIV binding function of a sCD-4 protein.

15. The process of claim 13 in which the vector is a S. cerevisiae vector in which the HIV binding protein is SKBsCD-4 or a derivative thereof having the HIV binding function of SKBsCD-4.

16. The process of claim 15 in which the HIV binding protein is selected from the group consisting of SKBsYCD-4 and YV1.

17. The process of claim 13, 14, 15 or 16 in which the regulatory element comprises a promoter and a leader sequence.

18. The process of claim 13, 14, 15 or 16 in which the HIV binding protein coding sequence is fused at its 5' end to the 3' end of a coding sequence which stabilizes expression of the HIV binding protein.

19. The process of claim 18 in which the coding sequence which stabilizes expression is the coding sequence for yeast ubiquitin.

20. The process of claim 19 in which the regulatory element is the CUP 1 promoter or the TDH3 promoter.